# EUROPEAN PATENT SPECIFICATION

(11) **EP 3 943 391 B1**
(45) Date of publication and mention of the grant of the patent: **06.08.2025**
(21) Application number: 21178757.7
(22) Date of filing: 10.06.2021
(51) Int. Cl.: B64D 11/00, A61L 2/10, B64D 11/04, B64F 5/30

(54) **SANITIZING GALLEY CART FOR AN INTERNAL CABIN OF A VEHICLE**
DESINFIZIERENDER BORDKÜCHENWAGEN FÜR DIE INNENKABINE EINES FAHRZEUGS
CHARIOT DE SERVICE D'ASSAINISSEMENT POUR CABINE INTERNE D'UN VÉHICULE

(30) Priority: 23.07.2020 US 202063055392 P; 21.04.2021 US 202117236178
(43) Date of publication of application: 26.01.2022
(73) Proprietor: The Boeing Company, Arlington, VA 22202 (US)
(72) Inventor: FREYLING, Brandon Wayne, Chicago, 60606-2016 (US)
(74) Representative: Boult Wade Tennant LLP

(56) References cited:
- WO-A1-2020/060507
- US-A1- 2014 059 796
- US-B2- 10 406 253

## Description

### FIELD OF THE DISCLOSURE

Examples of the present disclosure generally relate to sanitizing systems, such as may be used to sanitize structures and areas within vehicles, such as commercial aircraft.

### BACKGROUND OF THE DISCLOSURE

Vehicles such as commercial aircraft are used to transport passengers between various locations. Systems are currently being developed to disinfect or otherwise sanitize surfaces within aircraft, for example, that use ultraviolet (UV) light. However, many known UV light sanitizing systems are typically large, bulky, and often require fixed, stationary infrastructure.

US2014059796 A1, in accordance with its abstract, states systems and methods for disinfection of aircraft galleys and lavatories and other surfaces. UV light sources and/or a disinfection unit powered by a fuel cell or other external power source may be used. US2014059796 A1 also states, in accordance with its description, "... *there is provided a mobile, galley-sized cart that can project a UV light source on a surface to be treated. This ... provides a unit that can rolled out, disinfection can be conducted, and then the unit is rolled back into a galley cart storage area".*

### SUMMARY OF THE DISCLOSURE

A need exists for a system and a method for efficiently sterilizing surfaces within an internal cabin of a vehicle, such as a commercial aircraft.

There is described herein a galley cart for use within an internal cabin of a vehicle, the galley cart comprising: a base; wheels extending downwardly from the base; end walls extending upwardly from the base; lateral walls extending upwardly from the base; a top wall connected to the end walls and the lateral walls opposite from the base; an internal chamber defined between the base, the end walls, the lateral walls, and the top wall; and one or more ultraviolet (UV) light sources configured to emit UV light to sanitize one or more surfaces; wherein the one or more UV light sources are within the internal chamber; and wherein one or more of the base, the end walls, the lateral walls, and the top wall include one or more transparent portions that allow the UV light emitted by the one or more UV light sources to pass.

With that need in mind, certain examples of the present disclosure provide a galley cart for use within an internal cabin of a vehicle.

The galley cart may further include a mount coupled to the one or more UV light sources, and an actuator operatively coupled to the mount. The actuator is configured to move the one or more UV light sources between a stowed position within the internal chamber and an extended position outside of the internal chamber.

The one or more UV light sources may be secured to one or more exterior surfaces of one or more of the base, the end walls, the lateral walls, or the top wall.

The galley cart may not be configured to contain consumable items.

The galley cart can include a power source that is configured to provide power to the one or more UV light sources. The power source can include one or more batteries. The one or more batteries can be automatically recharged when the galley cart is stored in the cart compartment.

The galley cart can include a controller configured to control operation of the one or more UV light sources.

The galley cart may include a first compartment including the one or more UV light sources, and a second compartment configured to contain consumable items.

A galley insert may be removably contained within an insert compartment. The galley insert includes at least one UV light source.

The one or more UV light sources may be configured to emit the UV light at a wavelength of 222 nm.

There is also described herein a vehicle, optionally an aircraft, comprising: an internal cabin; a galley within the internal cabin; a cart compartment within the galley; and the galley cart described above, wherein the galley cart is configured to be stored within and removed from the cart compartment.

There is also described herein a method for sanitizing a surface of a component within an internal cabin of a vehicle, optionally an aircraft, the method comprising: providing a galley cart, the galley cart comprising: a base; wheels extending downwardly from the base; end walls extending upwardly from the base; lateral walls extending upwardly from the base; a top wall connected to the end walls and the lateral walls opposite from the base; an internal chamber defined between the base, the end walls, the lateral walls, and the top wall; and one or more ultraviolet (UV) light sources configured to emit UV light to sanitize one or more surfaces; wherein the one or more UV light sources are within the internal chamber; wherein one or more of the base, the end walls, the lateral walls, and the top wall include one or more transparent portions that allow the UV light emitted by the one or more UV light sources to pass; and wherein the galley cart is configured to be stored within a cart compartment within a galley of the internal cabin; and emitting UV light from the one or more UV light sources through the one or more transparent portions onto the surface of the component.

### BRIEF DESCRIPTION OF THE DRAWINGS

Figure 1 illustrates a perspective front view of an aircraft.
Figure 2A illustrates a top plan view of an internal cabin of an aircraft.
Figure 2B illustrates a top plan view of an internal cabin of an aircraft.
Figure 3 illustrates a perspective interior view of an internal cabin of an aircraft.
Figure 4 illustrate a lateral view of a galley cart.
Figure 5 illustrates a front view of the galley cart of Figure 4.
Figure 6 illustrates an internal view of the galley cart through line 6-6 of Figure 5.
Figure 7 illustrates a perspective view of a galley within an internal cabin.
Figure 8 illustrate a lateral view of a galley cart in accordance with the claims.
Figure 9 illustrates a front view of the galley cart of Figure 8.
Figure 10 illustrates an internal view of the galley cart through line 10-10 of Figure 9.
Figure 11 illustrates a front view of a galley cart that is not encompassed by the wording of the claims.
Figure 12 illustrates a lateral view of the galley cart of Figure 11.
Figure 13 illustrates a front view of a galley cart that is not encompassed by the wording of the claims.
Figure 14 illustrates a lateral view of a galley cart that is not encompassed by the wording of the claims.
Figure 15 illustrates a front view of the galley cart of Figure 14.
Figure 16 illustrates a perspective view of a galley insert in relation to an insert compartment within a galley.
Figure 17 illustrates an ultraviolet light spectrum.
Figure 18 illustrates a perspective end view of a UV light source.
Figure 19 illustrates a perspective end view of a UV light source.
Figure 20 illustrates a perspective end view of a UV light source.

### DETAILED DESCRIPTION OF THE DISCLOSURE

The foregoing summary, as well as the following detailed description of certain examples will be better understood when read in conjunction with the appended drawings. Further, references to "one example" are not intended to be interpreted as excluding the existence of additional examples that also incorporate the recited features. Moreover, unless explicitly stated to the contrary, examples "comprising" or "having" an element or a plurality of elements having a particular condition can include additional elements not having that condition.

The present disclosure provides a sanitizing system and method that includes an ultraviolet (UV) lamp (such as an excimer lamp) that emits UV light in a far UV light spectrum, such as at a wavelength of 222 nm, which neutralizes (such as kills) microbes (for example, viruses and bacteria), while posing no risk to humans. The UV lamp is used within an internal cabin to decontaminate and kill pathogens. The UV lamp is coupled to a galley cart that is configured for use within in a vehicle, such as a commercial aircraft

The present disclosure provides a galley cart configured for use within an internal cabin of a vehicle. The galley cart includes one or more UV light sources that are configured to emit UV light onto surfaces of components within the internal cabin to sanitize the surfaces.

By utilizing a frame of existing galley carts to include UV lighting systems, examples of the present disclosure provide portable sanitization systems that can be stored onboard a vehicle (such as a commercial aircraft), and moved throughout the internal cabin, such as between flights, during maintenance, and/or when the vehicle is in storage. The galley carts can be half carts, full carts, etc. Because the galley carts are already sized and shaped to be used and stored within internal cabins, they are easily used with existing vehicles, such as commercial aircraft. That is, there may be no need to provide separate and/or customized storage locations for the galley carts.

The present disclosure provides a galley cart for use within an internal cabin of a vehicle, such as a commercial aircraft. The galley cart includes one or more UV light sources, a power supply (such as one or more batteries), a controller, and at least one transparent wall. In at least one example, UV light from the UV light sources is emitted from all sides. In at least example, the galley cart has a first compartment for UV light disinfection, and a second compartment for storage of food, snacks, and/or beverages. In at least one example, the galley cart has a separate removeable self-contained UV light source.

The galley cart is configured to be stowed in a standard galley cart stowage compartment in an aircraft. The galley cart can be automatically charged when stowed.

The galley cart can include an extending arm that allows a UV light source to be extended beyond the interior of the galley cart. The extended arm can include a UV light emitting sphere, which allows the light to be emitted in all directions.

The galley cart can be stowed just like any other galley cart. An attendant can move the cart to a location that needs disinfecting. In at least one example, the UV light source(s) emit UV light at a wavelength of 222 nm, which is safe for use around humans, and therefore can be operated in the presence of individuals.

In at least one example, a self-contained UV light source can be contained in a galley insert, such as a container that locks into a specific location in a galley within an internal cabin. The insert sides can be transparent to allow the UV light to disperse from the container. Batteries can be charged while the insert is located in a cart compartment. Such charging can be accomplished via a blind mate power connect that is attached when the galley insert is pushed in and disconnects when the insert is removed. The galley insert can be removed from the cart compartment and placed where UV light disinfection is needed.

As described herein, the present disclosure provides a method for sanitizing a surface of a component within an internal cabin of a vehicle. The method includes providing a galley cart that is configured to be stored within a cart compartment within a galley of the internal cabin with one or more or more ultraviolet (UV) light sources. The method further includes emitting UV light from the UV light sources onto the surface.

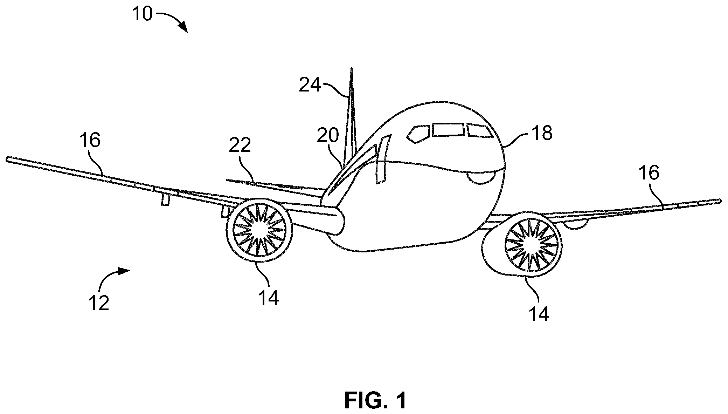

Figure 1 illustrates a perspective front view of an aircraft 10, according to an example of the present disclosure. The aircraft 10 includes a propulsion system 12 that includes engines 14, for example. Optionally, the propulsion system 12 may include more engines 14 than shown. The engines 14 are carried by wings 16 of the aircraft 10. In other examples, the engines 14 may be carried by a fuselage 18 and/or an empennage 20. The empennage 20 may also support horizontal stabilizers 22 and a vertical stabilizer 24.

The fuselage 18 of the aircraft 10 defines an internal cabin 30, which includes a flight deck or cockpit, one or more work sections (for example, galleys, personnel carry-on baggage areas, and the like), one or more passenger sections (for example, first class, business class, and coach sections), one or more lavatories, and/or the like. The internal cabin 30 includes one or more lavatory systems, lavatory units, or lavatories, as described herein.

Alternatively, instead of an aircraft, examples of the present disclosure may be used with various other vehicles, such as automobiles, buses, locomotives and train cars, watercraft, spacecraft, and the like. Further, examples of the present disclosure may be used with respect to fixed structures, such as commercial and residential buildings.

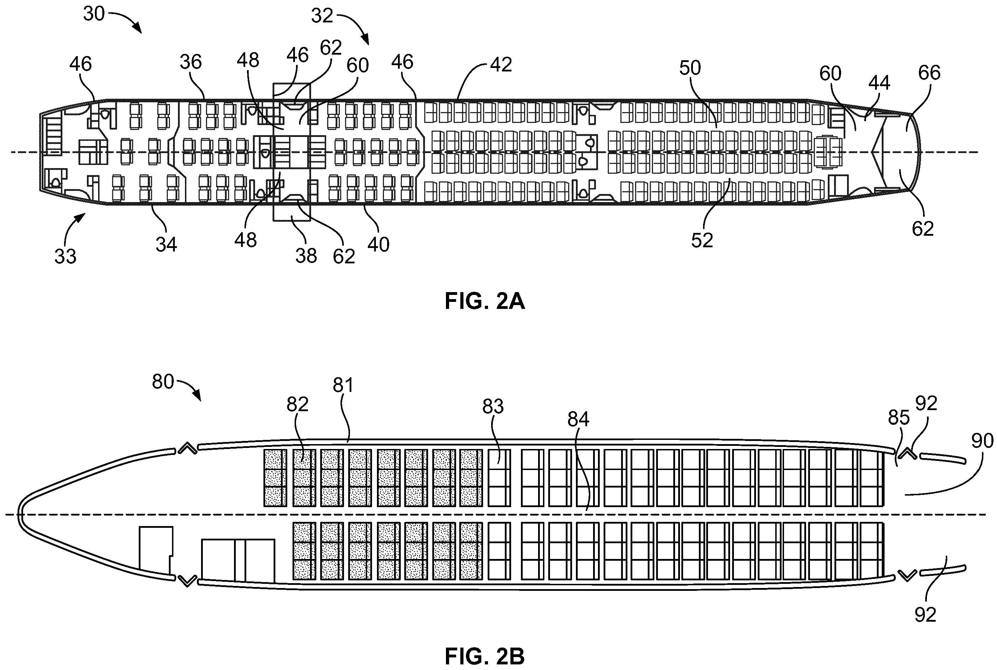

Figure 2A illustrates a top plan view of an internal cabin 30 of an aircraft, according to an example of the present disclosure. The internal cabin 30 may be within the fuselage 32 of the aircraft, such as the fuselage 18 of Figure 1. For example, one or more fuselage walls may define the internal cabin 30. The internal cabin 30 includes multiple sections, including a front section 33, a first class section 34, a business class section 36, a front galley 38, an expanded economy or coach section 40, a standard economy of coach section 42, and an aft section 44, which may include multiple lavatories and galleys. It is to be understood that the internal cabin 30 may include more or less sections than shown. For example, the internal cabin 30 may not include a first class section, and may include more or less galleys than shown. Each of the sections may be separated by a cabin transition area 46, which may include class divider assemblies between aisles 48.

As shown in Figure 2A, the internal cabin 30 includes two aisles 50 and 52 that lead to the aft section 44. Optionally, the internal cabin 30 may have less or more aisles than shown. For example, the internal cabin 30 may include a single aisle that extends through the center of the internal cabin 30 that leads to the aft section 44.

The aisles 48, 50, and 52 extend to egress paths or door passageways 60. Exit doors 62 are located at ends of the egress paths 60. The egress paths 60 may be perpendicular to the aisles 48, 50, and 52. The internal cabin 30 may include more egress paths 60 at different locations than shown. As described herein, lavatory systems may be located at or proximate to intersections of the aisles 48, 50, 52 and the egress paths 60.

Figure 2B illustrates a top plan view of an internal cabin 80 of an aircraft, according to an example of the present disclosure. The internal cabin 80 is an example of the internal cabin 30 shown in Figure 1. The internal cabin 80 may be within a fuselage 81 of the aircraft. For example, one or more fuselage walls may define the internal cabin 80. The internal cabin 80 includes multiple sections, including a main cabin 82 having passenger seats 83, and an aft section 85 behind the main cabin 82. It is to be understood that the internal cabin 80 may include more or less sections than shown.

The internal cabin 80 may include a single aisle 84 that leads to the aft section 85. The single aisle 84 may extend through the center of the internal cabin 80 that leads to the aft section 85. For example, the single aisle 84 may be coaxially aligned with a central longitudinal plane of the internal cabin 80.

The aisle 84 extends to an egress path or door passageway 90. Exit doors 92 are located at ends of the egress path 90. The egress path 90 may be perpendicular to the aisle 84. The internal cabin 80 may include more egress paths than shown. As described herein, lavatory systems may be located at or proximate to intersections of the aisle 84 and one or more egress paths 90.

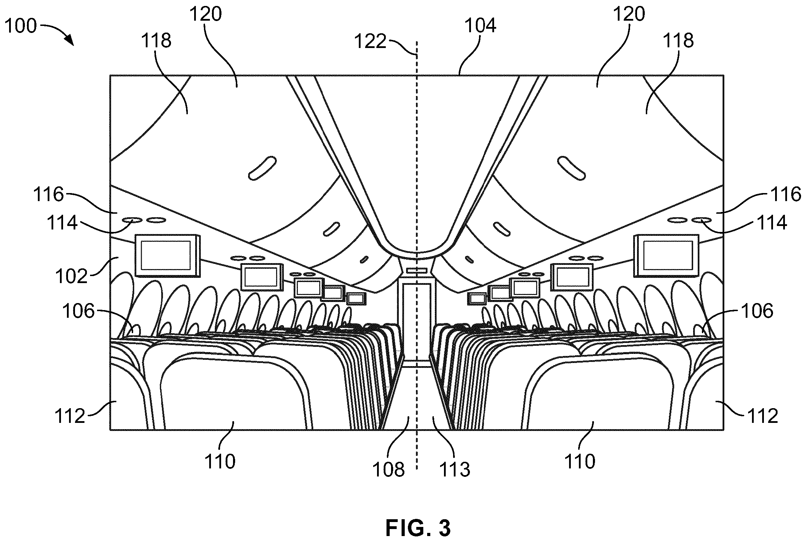

Figure 3 illustrates a perspective interior view of an internal cabin 100 of an aircraft, according to an example of the present disclosure. The internal cabin 100 is an example of the internal cabin 30, shown in Figure 1. The internal cabin 100 includes outboard walls 102 connected to a ceiling 104. Windows 106 may be formed within the outboard walls 102. A floor 108 supports rows of seats 110. As shown in Figure 3, a row 112 may include two seats 110 on either side of an aisle 113. However, the row 112 may include more or less seats 110 than shown. Additionally, the internal cabin 100 may include more aisles than shown.

Passenger service units (PSUs) 114 are secured between an outboard wall 102 and the ceiling 104 on either side of the aisle 113. The PSUs 114 extend between a front end and rear end of the internal cabin 100. For example, a PSU 114 may be positioned over each seat 110 within a row 112. Each PSU 114 may include a housing 116 that may contain vents, reading lights, an oxygen bag drop panel, an attendant request button, and/or other such controls over each seat 110 (or groups of seats) within a row 112.

Overhead stowage bin assemblies 118 are secured to the ceiling 104 and/or the outboard wall 102 above and inboard from the PSU 114 on either side of the aisle 113. The overhead stowage bin assemblies 118 are secured over the seats 110. The overhead stowage bin assemblies 118 extend between the front and rear end of the internal cabin 100. Each stowage bin assembly 118 may include a pivot bin or bucket 120 pivotally secured to a strongback (hidden from view in Figure 3). The overhead stowage bin assemblies 118 may be positioned above and inboard from lower surfaces of the PSUs 114. The overhead stowage bin assemblies 118 are configured to be pivoted open in order to receive passenger carry-on baggage and personal items, for example.

As used herein, the term "outboard" means a position that is further away from a central longitudinal plane 122 of the internal cabin 100 as compared to another component. The term "inboard" means a position that is closer to the central longitudinal plane 122 of the internal cabin 100 as compared to another component. For example, a lower surface of a PSU 114 may be outboard in relation to a stowage bin assembly 118.

Referring to Figures 1-3, galley carts are stored in cart compartments within galleys, such as the galley 38. The galley carts are sized and shaped to be stored within the cart compartments, removed therefrom, and move within the aisles of the internal cabin, such as the aisle 113. As described herein, at least one of the galley carts includes one or more UV light sources that are configured to emit UV light that sanitizes surfaces of components (such as seats, monuments, stowage bins, PSUs, floors, ceilings, and the like) within an internal cabin.

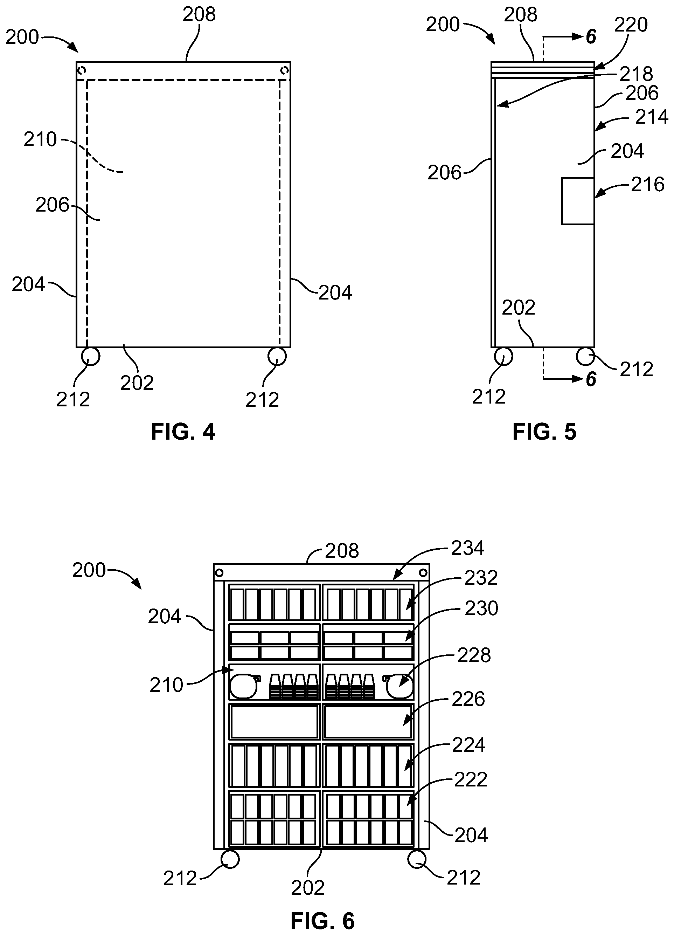

Figure 4 illustrate a lateral view of a galley cart 200, according to an example of the present disclosure. The galley cart 200 includes a base 202, end walls 204 extending upwardly from the base 202, lateral walls 206 extending upwardly from the base 202, and a counter or top wall 208 connected to the end walls 204 and the lateral walls 206 opposite from the base 202. An internal chamber 210 is defined between the base 202, the end walls 204, the lateral walls 206, and the top wall 208. Wheels 212 downwardly extend from the base 202. For example, the galley cart 200 includes four wheels 212 proximate to four corners of the base 202. The wheels 212 are configured to be supported on a floor of an internal cabin and allow the galley cart 200 to be rolled to different locations within the internal cabin.

Figure 5 illustrates a front view of the galley cart 200 of Figure 4. An end wall 204 at the front of the galley cart 200 includes a main door 214 that is configured to be selectively opened and closed. The main door 214 includes a latch handle 216 proximate to a first lateral wall 204, and is pivotally coupled to a hinge 218 proximate to a second lateral wall 204 that is opposite from the first lateral wall 204. The main door 214 is configured to pivotally open and close about the hinge 218. The galley cart 200 can also include a handle 220 extending from a top portion, such as at one or both ends.

Figure 6 illustrates an internal view of the galley cart 200 through line 6-6 of Figure 5. The internal chamber 210 of the galley cart 200 is typically configured to contain consumable items, such as beverages 222 (for example, soft drinks, cans of beer, bottles of liquor, and the like), water bottles 224, ice 226, coffee materials 228, prepared meals 230, snacks 232, and the like. Further, a work surface 234, such as a counter or ledge, can extend within and/or from the internal chamber 210 proximate to the top wall 208.

Referring to Figures 4-6, the galley cart 200 is sized and shaped to be contained or otherwise stored within a cart compartment within an internal cabin of a vehicle. The galley cart 200 is further sized and shaped to move into and through aisles of the internal cabin, such as the aisle 113 shown in Figure 3, which is between rows of seats. The galley cart 200 can be a full size cart, a half size cart, or the like. As described herein, the galley cart 200 includes one or more UV light sources that are configured to emit UV light to sanitize components within the internal cabin.

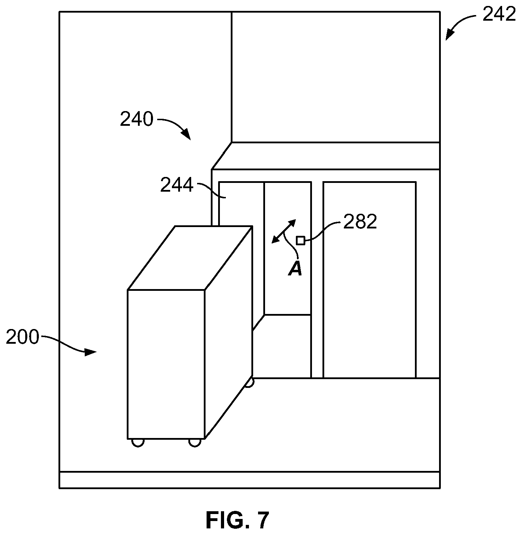

Figure 7 illustrates a perspective view of a galley 240 within an internal cabin 242, according to an example of the present disclosure. The galley 240 includes one or more cart compartments 244. A cart compartment 244 is configured to receive and retain the galley cart 200. For example, the galley cart 200 is configured to be moved into and out of the cart compartment 244 in the direction of arrows A.

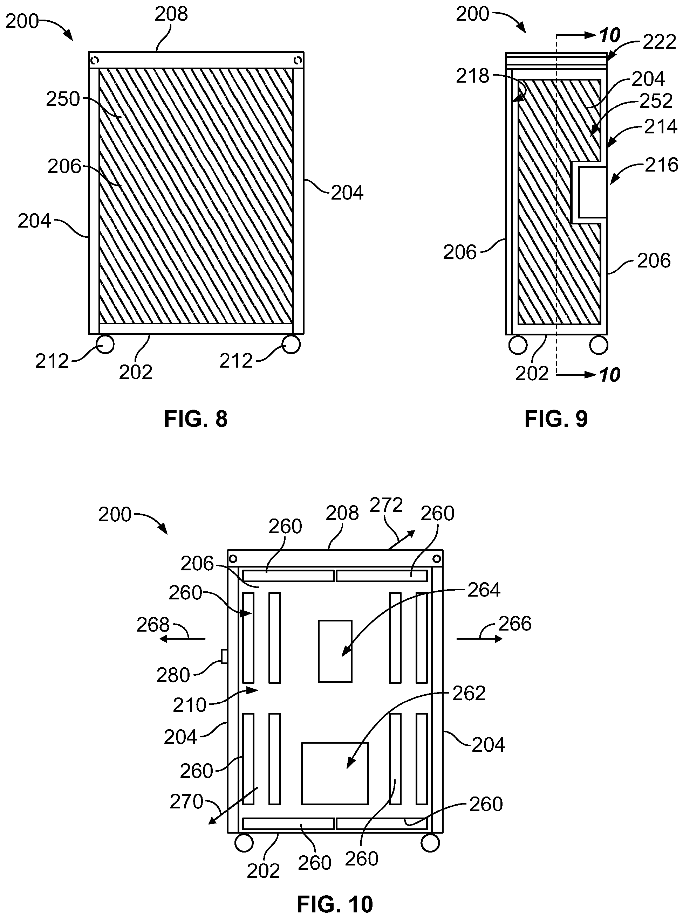

Figure 8 illustrate a lateral view of the galley cart 200, in accordance with the claims. One or both of the lateral walls 206 include transparent portions 250, such as panels, entire walls or sections thereof, and/or the like. For example, the transparent portions 250 may be or include glass or plexiglass panels that form the lateral walls 206, or portions thereof. The transparent portions 250 allow UV light emitted from UV light sources inside the galley cart 200 to pass therethrough. Optionally, in an example not encompassed by the wording of the claims, instead of the transparent portions 250, the lateral walls 206 can include outer frames with open spaces therebetween.

Figure 9 illustrates a front view of the galley cart 200 of Figure 8. One or both of the end walls 204 include transparent portions 252, such as panels, entire walls or sections thereof, and/or the like. For example, the transparent portions 252 may be or include glass or plexiglass panels that form the end walls 204, or portions thereof. The transparent portions 250 allow UV light emitted by UV light sources inside the galley cart 200 to pass therethrough. Optionally, in an example not encompassed by the wording of the claims, instead of the transparent portions 250, the end walls 204 can include outer frames with open spaces therebetween.

Figure 10 illustrates an internal view of the galley cart 200 through line 10-10 of Figure 9. As shown, one or more UV light sources 260 are secured within the internal chamber 210. In at least one example, the galley cart 200 does not contain consumable items. Instead, the galley cart 200 contains the UV light sources 260 and associated components, such as a power source 262 coupled to the UV light sources 260, and a controller 264 coupled to the UV light sources 260 (for example, one or more processors that are configured to control operation of the UV light sources 260). Because the UV light sources 260 are contained within the internal chamber 210, they are protected from being touched, bumped, inadvertently engaged, damaged, or the like from outside the galley cart 200.

Referring to Figures 8-10, the UV light sources 260 are configured to emit UV light through the transparent portions 250 and 252 to sanitize surfaces of components within an internal cabin. The internal chamber 210 contains UV light sources 260 to emit UV light in a forward direction 266, a rearward direction 268, a first lateral direction 270, and a second lateral direction 272, which is opposite the first lateral direction. That is, the UV light sources 260 are configured to emit the UV light from inside the internal chamber 210 around the galley cart 200. Optionally, the galley cart 200 can include UV light sources 260 that emit UV light in less than all of the forward direction 266, the rearward direction 268, the first lateral direction 270, and the second lateral direction 272.

In at least one example, the galley cart 200 includes one or more UV light sources 260 proximate to the top wall 208 (such as within 5 inches of less), one or more UV light sources 260 proximate to junctions of one or both end walls 204 and one or both lateral walls 206 (such as within 5 inches or less), one or more UV light sources 260 proximate to the base 202, and/or the like. One or more light sources 260 can be at or proximate to a center of the galley cart 200. The galley cart 200 can include more or less UV light sources 260 than shown.

As shown, the UV light sources 260 may be linear UV light arrays. As another option, one or more of the UV light sources 260 may be a spherical UV light source that emits UV light in all directions. For example, in at least one example, the galley cart 200 includes a single spherical UV light source 260 within the internal chamber 210. The UV light source 260 can emit UV light in all directions.

In at least one example, the top wall 208 and the base 202 can also include transparent portions. One or more UV light sources 260 can be configured to emit UV light through the top wall 208 and the base 202 to sanitize surfaces above and below the galley cart 200.

Referring to Figures 7-10, the controller 264 and the UV light sources 260 can be powered through a main power system within the internal cabin 242. For example, the galley cart 200 can include a plug 280 that is configured to dock with a reciprocal receptacle 282 (or vice versa) within the cart compartment 244 when the galley cart 200 is stored within the cart compartment 244. When the plug 280 is connected to the receptacle 282 (which is connected to a power source), electrical energy is transferred to the power source 262, such as one or more batteries, thereby automatically charging or recharging the batteries. In at least one other example, the power source 262 may be electrically connected to an electrical cord that includes a plug that is configured to removably couple to a reciprocal receptacle within the internal cabin.

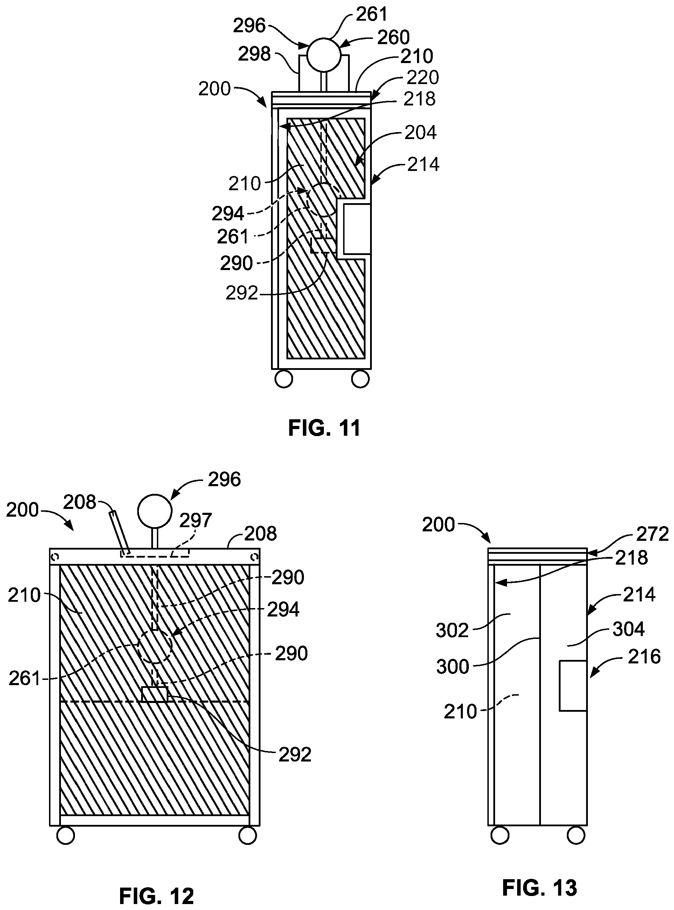

Figure 11 illustrates a front view of the galley cart 200, according to an example of the present disclosure. Figure 12 illustrates a lateral view of the galley cart 200 of Figure 11. Referring to Figures 11 and 12, in this example, a UV light source 260 may be or otherwise include a spherical UV lamp 261. The spherical UV lamp 261 can be fixed in position within the internal chamber 210 and configured to emit UV light in all radial directions.

In at least one other example, the UV light source 260 (whether the spherical UV lamp 261, a linear UV array, or the like) is coupled to a mount 290, such as a telescoping, articulating, pivoting, or the like arm(s) and/or beam(s), which, in turn is connected to an actuator 292, such as a motor. The actuator 292 is configured to move the UV light source 260 between a stowed position 294 within the internal chamber 210, and an extended position 296 outside of the internal chamber, such as the UV lamp 260 extending through an opening 297 within the top wall 208. For example, a door 298 may be opened to expose the opening 297. In at least one other example, the extended position is through one or more walls other than, and/or in addition to, the top wall 208.

Figure 13 illustrates a front view of the galley cart 200, according to an example of the present disclosure. In this example, the internal chamber 210 may include a dividing wall 300 that separates the internal chamber 210 into a first compartment 302 and a second compartment 304. The first compartment 302 contains one or more UV light sources 260, as shown and described with respect to Figures 8-13. The second compartment 304 is shielded from UV light by the dividing wall 300, and is configured to contains consumable items, as shown and described with respect to Figure 6.

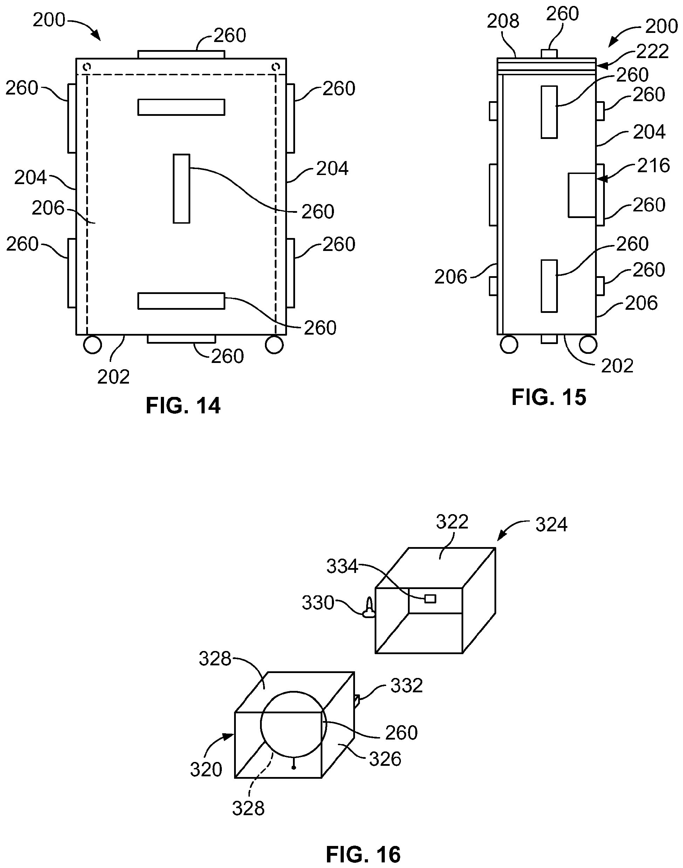

Figure 14 illustrates a lateral view of the galley cart 200, according to an example of the present disclosure. Figure 15 illustrates a front view of the galley cart 200 of Figure 14. Referring to Figures 14 and 15, in this example, one or more UV light sources 260 are secured to exterior surfaces of the one or more of the base 202, the end walls 204, the lateral walls 206, and/or the top wall 208. The galley cart 200 may or may not include transparent portions. More or less UV light sources 260 than shown may be used. Further, less than all of the base 202, the end walls 204, the lateral walls 206, and the top wall 208 may include UV light sources 260 on exterior surfaces thereof.

Figure 16 illustrates a perspective view of a galley insert 320 in relation to an insert compartment 322 within a galley 324, according to an example of the present disclosure. The galley insert 320 includes a container 326 defining an internal chamber 328. The container 326 is configured to be stored within the insert compartment 322. In at least one example, the galley insert 320 includes one or more UV light sources 260. For example, a spherical UV lamp 260 is within the internal chamber 328, and walls of the container 326 are transparent, thereby allowing UV light emitted by the UV lamp 260 to pass therethrough. Optionally, one or more UV light sources 260 can be linear arrays, and/or secured to external surfaces of the container 326, which may or may not include transparent walls.

When the galley insert 320 is in a stored position within the insert compartment 322, a latch 330 can be engaged to secure the galley insert 320 within insert compartment 322. Further, the galley insert 320 can include a plug 332 that mates with a reciprocal receptacle 334 within the insert compartment 322 so that a power source (for example, one or more batteries) of the galley insert 320 can be charged and re-charged.

The galley insert 320 can be removed from the insert compartment 322 and placed in a position within the internal cabin to sanitize surfaces of components. For example, the galley insert 320 can be positioned within a flight deck, seating area, lavatory, or the like within an internal cabin, and the UV light source 260 can be activated to emit light to sanitize various surfaces. Further, the galley insert 320 may be positioned on the galley cart 200 (such as shown in Figures 4-15).

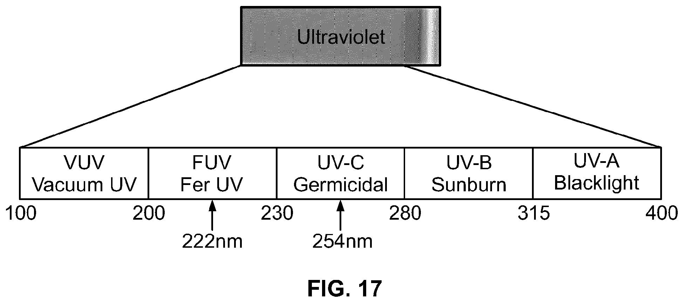

Figure 17 illustrates an ultraviolet light spectrum. Referring to Figures 4-17, the UV light sources 260 are configured to emit sanitizing UV light within a far UV spectrum, such as between 200 nm to 230 nm. In at least one example, the UV light sources 260 emit sanitizing UV light having a wavelength of 222 nm.

It has been found that sanitizing UV light having a wavelength of 222 nm kills pathogens (such as viruses and bacteria), instead of inactivating pathogens. In contrast, UVC light at a wavelength of 254 nm inactivates pathogens by interfering with their DNA, resulting in temporary inactivation, but may not kill the pathogens. Instead, the pathogen may be reactivated by exposure to ordinary white light at a reactivation rate of 10% per hour (or about 10% per hour). As such, UVC light at a wavelength of 254 nm may be ineffective in illuminated areas, such as within an internal cabin of a vehicle. Moreover, UVC light at 254 nm is not recommended for human exposure because it may be able to penetrate human cells.

In contrast, sanitizing UV light having a wavelength of 222 nm is safe for human exposure and kills pathogens. Further, the sanitizing UV light having a wavelength of 222 nm may be emitted at full power within one millisecond or less of a UV light source 260 being activated (in contrast the UVC light having a wavelength of 254 nm, which may take seconds or even minutes to reach full power).

Alternatively, the UV light sources 260 may emit sanitizing UV light at wavelengths other than between 220-230 nm. For example, the UV light sources 260 may emit sanitizing UV light between 230-280 nm, within the UVC spectrum.

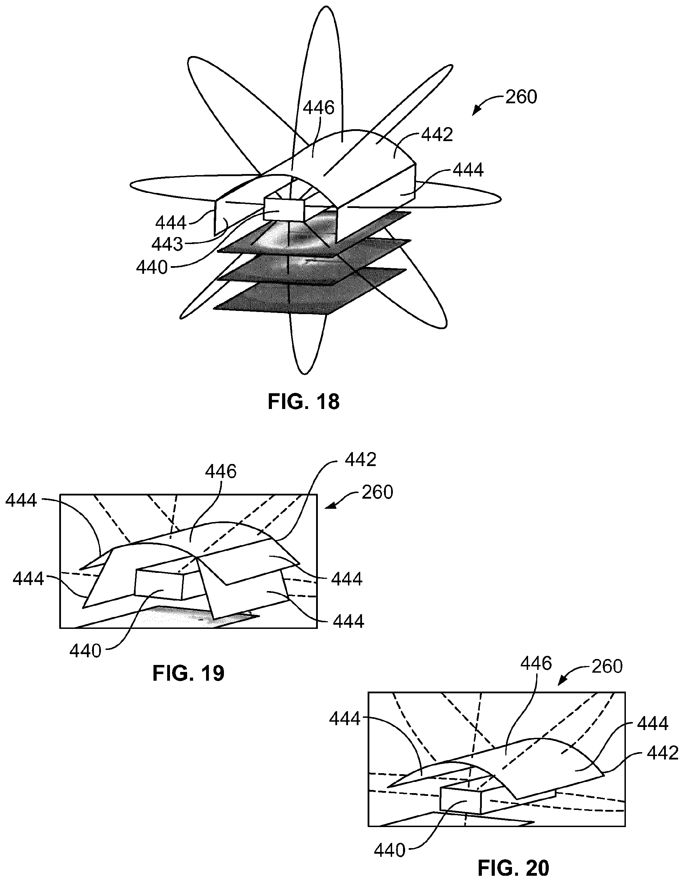

Figure 18 illustrates a perspective end view of a UV light source 260, according to an example of the present disclosure. The UV light source 260 shown in Figure 18 is merely exemplary. The UV light source 260 can be configured other than shown and described.

In at least one example, the UV light source 260 includes a UV lamp 440 and a reflector 442. The UV lamp 440 and the reflector 442 can be secured within a shroud, for example. In at least one example, the reflector 442 is secured to an underside of the shroud, such as through one or more adhesives. In at last one other example, the reflector 442 is an integral part of the shroud. For example, the reflector 442 may be or otherwise provide the underside of the shroud. The reflector 442 provides a reflective surface 443 (such as formed of Teflon, a mirrored surface, and/or the like) that is configured to outwardly reflect UV light emitted by the UV lamp 440. In at least one example, shroud may be or include a shell formed of fiberglass, and the reflector 442 may be formed of Teflon that provides a 98% reflectivity.

The reflector 442 may extend along an entire length of the underside of the shroud. Optionally, the reflector 442 may extend along less than an entire length of the underside of the shroud.

The UV lamp 440 may extend along an entire length (or along substantially the entire length). The UV lamp 440 is secured to the reflector 442 and/or the shroud through one or more brackets, for example. The UV lamp 440 includes one or more UV light emitters, such as one more bulbs, light emitting elements (such as light emitting diodes), and/or the like. In at least one example, the UV lamp 440 is configured to emit UV light in the far UV spectrum, such as at a wavelength between 200 nm - 230 nm. In at least one example, the UV lamp 440 is configured to emit UV light having a wavelength of 222 nm. For example, the UV lamp 440 may be or include a 300 W bulb that is configured to emit UV light having a wavelength of 222 nm.

As shown, the reflector 442 includes flat, upright side walls 444 connected together through an upper curved wall 446. The upper curved wall 446 may be bowed outwardly away from the UV lamp 440. For example, the upper curved wall 446 may have a parabolic cross-section and/or profile.

It has been found that the straight, linear side walls 444 provide desired reflection and/or focusing of UV light emitted from the UV lamp 440 toward and onto a desired location. Alternatively, the side walls 444 may not be linear and flat.

Figure 19 illustrates a perspective end view of the UV light source 260, according to an example of the present disclosure. The UV light source 260 shown in Figure 19 is merely exemplary. The UV light source 260 can be configured other than shown and described. The reflector 442 shown in Figure 19 is similar to the reflector 442 shown in Figure 18, except that the side walls 444 may outwardly cant from the upper curved wall 446.

Figure 20 illustrates a perspective end view of the UV light source 260, according to an example of the present disclosure. The UV light source 260 shown in Figure 20 is merely exemplary. The UV light source 260 can be configured other than shown and described. In this and other examples, the side walls 444 may be curved according to the curvature of the upper curved wall 446.

As described herein, examples of the present disclosure provide systems and a methods for efficiently sterilizing surfaces, components, structures, and/or the like within an internal cabin of a vehicle. Further, examples of the present disclosure provide compact, easy-to-use, and safe systems and methods for using UV light to sterilize surfaces within an internal cabin.

While various spatial and directional terms, such as top, bottom, lower, mid, lateral, horizontal, vertical, front and the like can be used to describe examples of the present disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

This written description uses examples to disclose the various examples of the disclosure, including the best mode, and also to enable any person skilled in the art to practice the various examples of the disclosure, including making and using any devices or systems and performing any incorporated methods. The patentable scope is defined by the claims, and can include other examples that occur to those skilled in the art.

## Claims

1. A galley cart (200) for use within an internal cabin (30, 80, 100, 242) of a vehicle, the galley cart (200) comprising:
a base (202);
wheels (212) extending downwardly from the base (202);
end walls (204) extending upwardly from the base (202);
lateral walls (206) extending upwardly from the base (202);
a top wall (208) connected to the end walls (204) and the lateral walls (206) opposite from the base (202);
an internal chamber (210) defined between the base (202), the end walls (204), the lateral walls (206), and the top wall (208); and
one or more ultraviolet (UV) light sources (260) configured to emit UV light to sanitize one or more surfaces;
wherein the one or more UV light sources (260) are within the internal chamber (210); and
wherein one or more of the base (202), the end walls (204), the lateral walls (206), and the top wall (208) include one or more transparent portions (250) that allow the UV light emitted by the one or more UV light sources (260) to pass.

2. The galley cart (200) of claim 1, wherein one of the end walls (204) comprises a door (214).

3. The galley cart (200) of claim 2, wherein the door (214) includes a latch handle (216) proximate a first of the lateral walls (204), and is pivotally coupled to a hinge (218) proximate to a second of the lateral walls (204) that is opposite from the first lateral wall (204).

4. The galley cart of claim 3, wherein the door (214) is configured to pivotally open and close about the hinge (281).

5. The galley cart (200) of any preceding claim, further comprising:
a mount (290) coupled to the one or more UV light sources (260); and
an actuator (292) operatively coupled to the mount (290), wherein the actuator (292) is configured to move the one or more UV light sources (260) between a stowed position (294) within the internal chamber (210) and an extended position (296) outside of the internal chamber (210).

6. The galley cart (200) of any preceding claim, wherein the one or more UV light sources (260) are secured to one or more exterior surfaces of one or more of the base (202), the end walls (204), the lateral walls (206), or the top wall (208).

7. The galley cart (200) of any preceding claim, further comprising a power source (262) that is configured to provide power to the one or more UV light sources (260).

8. The galley cart (200) of claim 7, wherein the power source (262) comprises one or more batteries.

9. The galley cart (200) of claim 8, wherein the one or more batteries are configured to be automatically recharged when the galley cart is stored in a cart compartment.

10. The galley cart (200) of any preceding claim, further comprising a controller (264) configured to control operation of the one or more UV light sources (260).

11. The galley cart (200) of any preceding claim, further comprising:
a first compartment (302) including the one or more UV light sources (260); and
a second compartment (304) configured to contain consumable items.

12. The galley cart (200) of any preceding claim, further comprising a galley insert (320) removably contained within an insert compartment (322), wherein the galley insert (320) comprises the one or more UV light sources (260).

13. The galley cart (200) of any preceding claim, wherein the one or more UV light sources (260) are configured to emit the UV light at a wavelength of 222 nm.

14. A vehicle, optionally an aircraft (10), comprising:
an internal cabin (30, 80, 100, 242);
a galley (324) within the internal cabin (30, 80, 100, 242);
a cart compartment (244) within the galley (324); and
the galley cart (200) of any preceding claim, wherein the galley cart (200) is configured to be stored within and removed from the cart compartment (244).

15. A method for sanitizing a surface of a component within an internal cabin (30, 80, 100, 242) of a vehicle, optionally an aircraft (10), the method comprising:
providing a galley cart (200), the galley cart (200) comprising:
a base (202);
wheels (212) extending downwardly from the base (202);
end walls (204) extending upwardly from the base (202);
lateral walls (206) extending upwardly from the base (202);
a top wall (208) connected to the end walls (204) and the lateral walls (206) opposite from the base (202);
an internal chamber (210) defined between the base (202), the end walls (204), the lateral walls (206), and the top wall (208); and
one or more ultraviolet (UV) light sources (260) configured to emit UV light to sanitize one or more surfaces;
wherein the one or more UV light sources (260) are within the internal chamber (210);
wherein one or more of the base (202), the end walls (204), the lateral walls (206), and the top wall (208) include one or more transparent portions (250) that allow the UV light emitted by the one or more UV light sources (260) to pass; and
wherein the galley cart (200) is configured to be stored within a cart compartment (244) within a galley (324) of the internal cabin (30, 80, 100, 242); and
emitting UV light from the one or more UV light sources (260) through the one or more transparent portions (250) onto the surface of the component.

## Patentansprüche

1. Bordküchenwagen (200) zur Verwendung in einer Innenkabine (30, 80, 100, 242) eines Verkehrsmittels, wobei der Küchenwagen (200) umfasst:
eine Basis (202);
Räder (212), die sich von der Basis (202) nach unten erstrecken;
Stirnwände (204), die sich von der Basis (202) nach oben erstrecken;
Seitenwände (206), die sich von der Basis (202) nach oben erstrecken;
eine Deckwand (208), die mit den Stirnwänden (204) und den Seitenwänden (206) gegenüber der Basis (202) verbunden ist;
eine Innenkammer (210), die zwischen der Basis (202), den Stirnwänden (204), den Seitenwänden (206) und der Deckwand (208) definiert ist; und
eine oder mehrere UV-Lichtquellen (260), die konfiguriert sind, um UV-Licht zum Desinfizieren von einer oder mehrere Oberflächen zu emittieren;
wobei die ein oder mehreren UV-Lichtquellen (260) sich in der Innenkammer (210) befinden; und
wobei eine oder mehrere von Basis (202), Stirnwänden (204), Seitenwänden (206) und Deckwand (208) einen oder mehrere transparente Abschnitte (250) aufweisen, die das von der einen oder den mehreren UV-Lichtquellen (260) emittierte UV-Licht durchlassen.

2. Bordküchenwagen (200) nach Anspruch 1, bei dem eine der Stirnwände (204) eine Tür (214) umfasst.

3. Bordküchenwagen (200) nach Anspruch 2, bei dem die Tür (214) einen Verriegelungsgriff (216) in der Nähe einer ersten der Seitenwände (204) umfasst und schwenkbar mit einem Scharnier (218) in der Nähe einer zweiten der Seitenwände (204) verbunden ist, die der ersten Seitenwand (204) gegenüberliegt.

4. Bordküchenwagen nach Anspruch 3, bei dem die Tür (214) zum um das Scharnier (281) schwenkenden Öffnen und Schließen konfiguriert ist.

5. Bordküchenwagen (200) nach einem der vorhergehenden Ansprüche, ferner umfassend:
eine mit den ein oder mehreren UV-Lichtquellen (260) verbundene Halterung (290); und
einen Aktuator (292), der funktionsmäßig mit der Halterung (290) gekoppelt ist, wobei der Aktuator (292) konfiguriert ist, um die eine oder die mehreren UV-Lichtquellen (260) zwischen einer verstauten Position (294) in der inneren Kammer (210) und einer ausgefahrenen Position (296) außerhalb der inneren Kammer (210) zu bewegen.

6. Bordküchenwagen (200) nach einem der vorhergehenden Ansprüche, bei dem die ein oder mehreren UV-Lichtquellen (260) an einer oder mehreren Außenflächen der Basis (202), der Stirnwände (204), der Seitenwände (206) oder der Deckwand (208) befestigt sind.

7. Bordküchenwagen (200) nach einem der vorhergehenden Ansprüche, ferner umfassend eine Energiequelle (262), die konfiguriert ist, um die eine oder die mehreren UV-Lichtquellen (260) mit Energie zu versorgen.

8. Bordküchenwagen (200) nach Anspruch 7, bei dem die Energiequelle (262) eine oder mehrere Batterien umfasst.

9. Bordküchenwagen (200) nach Anspruch 8, bei dem die ein oder mehreren Batterien konfiguriert sind, um automatisch wieder aufgeladen werden, wenn der Bordküchenwagen in einem Wagenfach gelagert ist.

10. Bordküchenwagen (200) nach einem der vorhergehenden Ansprüche, ferner umfassend eine Steuerung (264), die konfiguriert ist, um den Betrieb der ein oder mehreren UV-Lichtquellen (260) zu steuern.

11. Bordküchenwagen (200) nach einem der vorhergehenden Ansprüche, ferner umfassend:
ein erstes Fach (302) mit den ein oder mehreren UV-Lichtquellen (260); und
ein zweites Fach (304), das konfiguriert ist, um verzehrbare Gegenstände aufzunehmen.

12. Bordküchenwagen (200) nach einem der vorhergehenden Ansprüche, ferner umfassend einen Wageneinsatz (320), der entfernbar in einem Einsatzfach (322) enthalten ist, wobei der Wageneinsatz (320) die ein oder mehreren UV-Lichtquellen (260) umfasst.

13. Bordküchenwagen (200) nach einem der vorhergehenden Ansprüche, wobei die ein oder mehreren UV-Lichtquellen (260) konfiguriert sind, um das UV-Licht mit einer Wellenlänge von 222 nm zu emittieren.

14. Verkehrsmittel, optional ein Flugzeug (10), umfassend:
eine Innenkabine (30, 80, 100, 242);
eine Bordküche (324) in der Innenkabine (30, 80, 100, 242);
ein Wagenfach (244) in der Bordküche (324); und
den Bordküchenwagen (200) nach einem der vorhergehenden Ansprüche, wobei der Bordküchenwagen (200) konfiguriert ist, um in dem Wagenfach (244) aufbewahrt und aus diesem entnommen zu werden.

15. Verfahren zum Desinfizieren einer Oberfläche einer Komponente in einer Innenkabine (30, 80, 100, 242) eines Verkehrsmittels, optional eines Flugzeugs (10), wobei das Verfahren umfasst:
Bereitstellen eines Bordküchenwagens (200), wobei der Bordküchenwagen (200) umfasst:
eine Basis (202);
Räder (212), die sich von der Basis (202) nach unten erstrecken;
Stirnwände (204), die sich von der Basis (202) nach oben erstrecken;
Seitenwände (206), die sich von der Basis (202) nach oben erstrecken;
eine Deckwand (208), die mit den Stirnwänden (204) und den Seitenwänden (206) gegenüber der Basis (202) verbunden ist;
eine Innenkammer (210), die zwischen der Basis (202), den Stirnwänden (204), den Seitenwänden (206) und der Deckwand (208) definiert ist; und
eine oder mehrere Ultraviolettlichtquellen (UV) (260), die konfiguriert sind, um UV-Licht zum Desinfizieren einer oder mehrerer Oberflächen zu emittieren;
wobei die ein oder mehreren UV-Lichtquellen (260) sich in der Innenkammer (210) befinden;
wobei eine oder mehrere von Basis (202), Stirnwänden (204), Seitenwänden (206) und Deckwand (208) einen oder mehrere transparente Abschnitte (250) aufweisen, die das von den ein oder mehreren UV-Lichtquellen (260) emittierte UV-Licht durchlassen; und
wobei der Bordküchenwagen (200) konfiguriert ist, um in einem Wagenfach (244) in einer Bordküche (324) der Innenkabine (30, 80, 100, 242) aufbewahrt zu werden; und
Emittieren von UV-Licht von den ein oder mehreren UV-Lichtquellen (260) durch die ein oder mehreren transparenten Abschnitte (250) auf die Oberfläche der Komponente.

## Revendications

1. Chariot d'office (200) pour une utilisation à l'intérieur d'une cabine interne (30, 80, 100, 242) d'un véhicule, le chariot d'office (200) comprenant :
une base (202) ;
des roues (212) s'étendant vers le bas à partir de la base (202) ;
des parois d'extrémité (204) s'étendant vers le haut à partir de la base (202) ;
des parois latérales (206) s'étendant vers le haut à partir de la base (202) ;
une paroi supérieure (208) reliée aux parois d'extrémité (204) et aux parois latérales (206) à l'opposé de la base (202) ;
une chambre interne (210) définie entre la base (202), les parois d'extrémité (204), les parois latérales (206) et la paroi supérieure (208) ; et
une ou plusieurs sources de lumière ultraviolette (UV) (260) configurées pour émettre une lumière UV afin de désinfecter une ou plusieurs surfaces ;
dans lequel les une ou plusieurs sources de lumière UV (260) sont à l'intérieur de la chambre interne (210) ; et
dans lequel une ou plusieurs parmi la base (202), les parois d'extrémité (204), les parois latérales (206) et la paroi supérieure (208) comprennent une ou plusieurs parties transparentes (250) qui permettent à la lumière UV émise par les une ou plusieurs sources de lumière UV (260) de passer.

2. Chariot d'office (200) selon la revendication 1, dans lequel une certaine des parois d'extrémité (204) comprend une porte (214).

3. Chariot d'office (200) selon la revendication 2, dans lequel la porte (214) inclut une poignée de verrouillage (216) à proximité d'une première des parois latérales (204), et est couplée de manière pivotante à une charnière (218) à proximité d'une seconde des parois latérales (204) qui est opposée à la première paroi latérale (204).

4. Chariot d'office selon la revendication 3, dans lequel la porte (214) est configurée pour s'ouvrir et se fermer de manière pivotante autour de la charnière (281).

5. Chariot d'office (200) selon l'une quelconque des revendications précédentes, comprenant en outre :
un support (290) couplé aux une ou plusieurs sources de lumière UV (260) ; et
un actionneur (292) couplé de manière opérationnelle au support (290), dans lequel l'actionneur (292) est configuré pour déplacer les une ou plusieurs sources de lumière UV (260) entre une position rangée (294) à l'intérieur de la chambre interne (210) et une position étendue (296) à l'extérieur de la chambre interne (210).

6. Chariot d'office (200) selon l'une quelconque des revendications précédentes, dans lequel les une ou plusieurs sources de lumière UV (260) sont fixées à une ou plusieurs surfaces extérieures d'une ou plusieurs parmi la base (202), les parois d'extrémité (204), les parois latérales (206) ou la paroi supérieure (208).

7. Chariot d'office (200) selon l'une quelconque des revendications précédentes, comprenant en outre une source d'alimentation (262) qui est configurée pour fournir de la puissance aux une ou plusieurs sources de lumière UV (260).

8. Chariot d'office (200) selon la revendication 7, dans lequel la source d'alimentation (262) comprend une ou plusieurs batteries.

9. Chariot d'office (200) selon la revendication 8, dans lequel les une ou plusieurs batteries sont configurées pour être rechargées automatiquement lorsque le chariot d'office est stocké dans un compartiment de chariot.

10. Chariot d'office (200) selon l'une quelconque des revendications précédentes, comprenant en outre un dispositif de commande (264) configuré pour commander le fonctionnement des une ou plusieurs sources de lumière UV (260).

11. Chariot d'office (200) selon l'une quelconque des revendications précédentes, comprenant en outre :
un premier compartiment (302) incluant les une ou plusieurs sources de lumière UV (260) ; et
un second compartiment (304) configuré pour contenir des éléments consommables.

12. Chariot d'office (200) selon l'une quelconque des revendications précédentes, comprenant en outre un insert d'office (320) contenu de manière amovible dans un compartiment d'insert (322), dans lequel l'insert d'office (320) comprend les une ou plusieurs sources de lumière UV (260).

13. Chariot d'office (200) selon l'une quelconque des revendications précédentes, dans lequel les une ou plusieurs sources de lumière UV (260) sont configurées pour émettre la lumière UV à une longueur d'onde de 222 nm.

14. Véhicule, facultativement un aéronef (10), comprenant :
une cabine interne (30, 80, 100, 242) ;
un office (324) à l'intérieur de la cabine interne (30, 80, 100, 242) ;
un compartiment de chariot (244) à l'intérieur de l'office (324) ; et
le chariot d'office (200) selon l'une quelconque des revendications précédentes, dans lequel le chariot d'office (200) est configuré pour être stocké dans et retiré du compartiment de chariot (244).

15. Procédé pour désinfecter une surface d'un composant à l'intérieur d'une cabine interne (30, 80, 100, 242) d'un véhicule, facultativement d'un aéronef (10), le procédé comprenant les étapes consistant à :
fournir un chariot d'office (200), le chariot d'office (200) comprenant :
une base (202) ;
des roues (212) s'étendant vers le bas à partir de la base (202) ;
des parois d'extrémité (204) s'étendant vers le haut à partir de la base (202) ;
des parois latérales (206) s'étendant vers le haut à partir de la base (202) ;
une paroi supérieure (208) reliée aux parois d'extrémité (204) et aux parois latérales (206) à l'opposé de la base (202) ;
une chambre interne (210) définie entre la base (202), les parois d'extrémité (204), les parois latérales (206) et la paroi supérieure (208) ; et
une ou plusieurs sources de lumière ultraviolette (UV) (260) configurées pour émettre une lumière UV afin de désinfecter une ou plusieurs surfaces ;
dans lequel les une ou plusieurs sources de lumière UV (260) sont à l'intérieur de la chambre interne (210) ;
dans lequel une ou plusieurs parmi la base (202), les parois d'extrémité (204), les parois latérales (206) et la paroi supérieure (208) incluent une ou plusieurs parties transparentes (250) qui permettent à la lumière UV émise par les une ou plusieurs sources de lumière UV (260) de passer ; et
dans lequel le chariot d'office (200) est configuré pour être stocké à l'intérieur d'un compartiment de chariot (244) à l'intérieur d'un office (324) de la cabine interne (30, 80, 100, 242) ; et
émettre de la lumière UV à partir des une ou plusieurs sources de lumière UV (260) à travers les une ou plusieurs parties transparentes (250) jusque sur la surface du composant.
